# EUROPEAN PATENT SPECIFICATION

(11) **EP 2 578 266 B1**
(45) Date of publication and mention of the grant of the patent: **01.08.2018**
(21) Application number: 12157265.5
(22) Date of filing: 28.02.2012
(51) Int. Cl.: A61N 1/06, A61B 18/18

(54) **High-frequency electro medical device**
Elektro-medizinisches Hochfrequenzgerät
Dispositif électromédical d'haute fréquence

(30) Priority: 07.10.2011 IT PD20110318
(43) Date of publication of application: 10.04.2013
(73) Proprietor: Focusmed S.r.l., 35036 Montegrotto Terme (PD) (IT)
(72) Inventor: Martinangeli, Roberto, 35036 Montegrotto Terme (PD) (IT); Martinangeli, Carlo, 35036 Montegrotto Terme (PD) (IT)
(74) Representative: Vinci, Marcello

(56) References cited:
- US-A1- 2003 220 635
- US-A1- 2004 030 334
- US-A1- 2007 088 413
- US-A1- 2009 018 628
- US-A1- 2009 281 537
- US-A1- 2011 015 687
- US-B1- 7 758 537

## Description

The present invention concerns those electro medical devices that emit high-frequency magnetic fields, and in particular it concerns a new high-frequency electro medical device for pain therapy and physiotherapy in general..

High-frequency electro medical devices are known which generate an electromagnetic field and in which, with the aid of contact accessories, the high-frequency wave passes through the epidermis, the dermis and the hypodermis and reaches the muscle layer beneath the skin, stimulating the biological muscular and connective tissue.

The in-depth stimulation of the tissues obtained in this way has several positive effects. In fact, the use of said electro medical devices is known for many applications.

For example, it is known that high-frequency electro medical devices are used for the treatment of some pathologies affecting the muscle-tendon system.

Electro medical devices for pain therapy are known, used for example for the treatment of pain with rheumatic origin, chronic and acute backache, chronic cervical pain, periarthritis, arthrosis, herniated disks, joint sprains, contusions, stretches etc.

Said electro medical devices operate generating low-power and high-frequency pulsed electric fields that, passing through the body's tissues, cause the stimulation of the dermal and muscle cells down to the bones.

Said devices comprise a high-frequency wave generator connected to a hand piece to be applied to the patient's body, at the level of the area to be treated.

The treatment must be repeated in more than one session according to predefined time intervals and programmed cycles. US-A1-2007/088413 discloses a high-frequency electro medical device comprising a high-frequency generator connected to a power supply network, a hand piece with an application head, wherein the generator emits waves at pre-established operation frequencies (range from several hundreds KHz to 20 MHz), the surface of application head being a plate subjected to surface vitrification, and an insulated return mat electrically connected to the generator to close the high-frequency current circuit.

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. The object of the present invention is a new type of high-frequency electro medical device for pain therapy, physiotherapy and cell stimulation in general.

The main object of the present invention is to promote the stimulation of skin and muscle tissues while at the same time ensuring maximum adaptability to the therapy to be administered.

The present invention can be effectively applied in particular for the treatment of pain caused by a wide variety of pathologies, thus developing also an important anti inflammatory and anti oedematous action. For example, it is possible to treat pain caused by joint sprains, muscle stretches and lacerations, bruises, inflammation, contractures, tendinitis, bursitis, arthrosis etc.

An advantage offered by the invention, in fact, lies in that it is possible to select the frequency, power, duration and type of the treatment cycle, as well as other functions.

A further advantage offered by the present invention lies in that it is possible to use two types of hand pieces in order to have different application powers available with no need to vary the operating parameters of the device.

Another advantage of the present invention lies in that it guarantees the best safety conditions for the patient, since it is provided with suitable protection devices.

A further advantage of the present invention lies in that it is simple and immediate to use and no additional mechanical procedure is required.

These and other direct and complementary objects are achieved by the new high-frequency electro medical device for pain therapy, physiotherapy and cell stimulation in general, whose main parts comprise:
- at least one high-frequency generator suited to be connected to the power supply network;
- one or preferably at least two hand pieces suited to be connected to said generator by means of an electric cable;
- at least one or more application heads suited to be installed on said hand pieces,
and wherein said generator emits waves in two or more possible operating frequencies, according to the type of treatment to be made and to the patient.

In particular, the generator can emit 2 MHz, 4 MHz and 8 MHz waves.

For example, 2 MHz waves are particularly suitable for treating elderly people, while 8 MHz waves are particularly suitable for achieving anti inflammatory effects.

The generator can emit waves with adjustable emission power, with an emission cycle having adjustable duration, and the repetition or repetitions of said cycle are programmable.

The maximum emission of the generator is preferably continuous at 100 W rms, on 100 Ohm.

In the preferred embodiment of the invention said generator comprises at least one power supply switch and is connected to at least one control panel for the selection of said operating parameters and for the display of the operating status.

Said control panel preferably comprises a display, for example a touch screen display.

Said generator can be connected to the power supply network at 110-220V and at 50/60 Hz, with a maximum power absorption of 250 VA.

In the preferred embodiment of the invention, said device comprises at least two of said hand pieces, each provided with a corresponding, substantially plate-like application head, for example circular in shape, wherein said application heads of the two hand pieces come in different sizes.

Therefore, the same power being emitted, and the other operating parameters being the same, it is possible to carry out stimulation with different intensity, depending on which hand piece is used.

In particular, if for example the hand piece with smaller application head is used, the application intensity will be higher than the application intensity obtained using the hand piece with larger application head.

In the preferred embodiment of the invention, said device comprises a first hand piece with a 40 mm application head, for example, and maximum intensity of approximately 8 Jcm2 during normal use, and a second hand piece with a 50 mm application head, for example, and maximum intensity of approximately 5 Jcm2.

According to the invention, the new device may also comprise a further larger hand piece, for example with a 60 mm application head and maximum intensity of 3,5 Jcm2.

Said plates are integrally constrained to the hand piece, for example by screwing, so that they can be easily removed when they have to be replaced.

The surface of said plates is subjected to a surface vitrification treatment, for example with epoxy paints baked at 250°C, and they can be, for example, white.

This embodiment allows immediate check of the plate wear condition. In fact, the operator can immediately be aware that the plates need to be replaced if he/she notices abrasions and/or colour changes on their surface.

Said surface treatment of said plates furthermore protects the plates from the use of normal sterilizing and hygienizing products.

The new device also comprises at least one insulated return mat suited to be electrically connected to said generator in order to close the high-frequency current circuit.

Said mat must be placed between the patient and the supporting surface, chair or bed where the patient is positioned.

The new device preferably comprises also a containing case or body suited to house said generator, said hand piece and the attached accessories.

In order to use the new device correctly, it is necessary to:
- connect the generator to the power supply network;
- position said insulated return mat and connect it to said generator;
- connect at least one of said hand pieces to said generator;
- activate the generator;
- select the operating parameters: frequency, emission power, duration of the unit emission cycle, cycle repetition plan;
- position said plate of the hand piece on the tissues to be treated and maintain it as adherent to the tissues as possible, in order to keep emission constant; the emission varies from a minimum to a maximum value, depending on the parameters set;
- keep said plate of the hand piece in contact with the tissues for the whole duration of the cycle and for all the successive cycles.

The emission cycle, for example, is included between 1 and 30 minutes, for a maximum number of twenty repetitions.

The new device operates with suitable safety measures.

According to the invention, for example, the new device excludes the activation of the generator in case of incorrect connection or lack of connection between the generator and the hand piece.

Furthermore, the generator operates at minimum power until the plate has come into contact with the patient's tissues.

The generator operates at minimum power also in case of incorrect connection or lack of connection between said generator and said insulated return mat.

Furthermore, the generator is automatically and immediately deactivated if one or more power supply circuit/circuits is/are out of control and/or if the power amplifier absorption exceeds the maximum allowable current.

The characteristics of the new device will be highlighted in greater detail in the following description with reference to the attached drawings, which are provided by way of example without limitation.

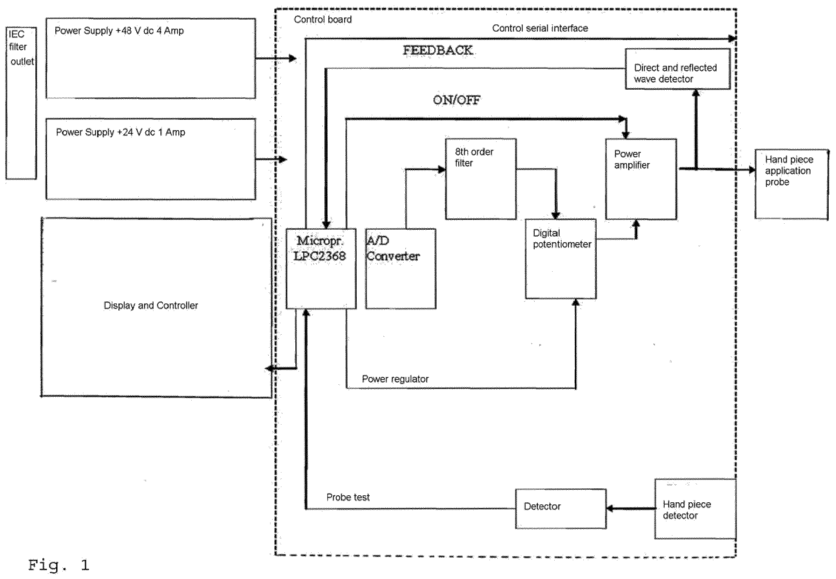

Figure 1 shows an operating diagram of the new device.

Therefore, with reference to the above description and the attached drawing, the following claims are expressed.

## Claims

1. High-frequency electro medical device for pain therapy and physiotherapy, emitting waves at two or more possible discrete and pre-established operation frequencies, activate the cell stimulation in muscle and connective tissue, comprising at least one high-frequency generator suited to be connected to a power supply network, at least one hand piece with application head suited to be connected to said generator, said generator, according to the type of treatment to be carried out and to the patient, wherein said generator is configured so that it cannot emit waves at values different from the pre-established operation frequencies of 2 MHz 4 MHz and 8 MHz, selecting one frequency at a time, with adjustable emission power, with an emission cycle having adjustable duration, and the repetition or repetitions of said cycle are programmable, and wherein said electro-medical device comprises:
• at least one or two of said hand pieces, each provided with a corresponding, substantially plate-like application head, and wherein said application heads of the two hand pieces come in different sizes, the surface of said plate being subjected to a surface vitrification treatment;
• at least one insulated return mat, suited to be electrically connected to said generator in order to close the high-frequency current circuit, and wherein said mat must be placed between the patient and the supporting surface, chair or bed where the patient is positioned;
and wherein the maximum power output of the generator is substantially continuous of 100 W rms on a load substantially on 100 Ohm.

2. High-frequency electro medical device according to any of the preceding claims, **characterized in that**, with the same power emission, said device carries out stimulation with different intensity, depending on the hand piece used.

3. High-frequency electro medical device according to any of the preceding claims, **characterized in that** said device comprises a first hand piece with a 40 mm application head and maximum intensity of approximately 8 Jcm2 during normal use, and a second hand piece with a 50 mm application head and maximum intensity of approximately 5 Jcm2.

4. High-frequency electro medical device according to any of the preceding claims, **characterized in that** it also comprises a further hand piece with a 60 mm application head and intensity of approximately 3,5 Jcm2.

5. High-frequency electro medical device according to any of the preceding claims, **characterized in that** said plate of each hand piece is removably constrained to the same hand piece.

6. High-frequency electro medical device according to any of the preceding claims, **characterized in that** it excludes the activation of the generator in case of incorrect connection or lack of connection between the generator and the hand piece.

7. High-frequency electro medical device according to any of the preceding claims, **characterized in that** said generator operates with minimum power when said plate of the hand piece is not in contact with the patient's tissue and/or in case of incorrect connection or lack of connection between said generator and said insulated return mat.

8. High-frequency electro medical device according to any of the preceding claims, **characterized in that** said generator is automatically and immediately deactivated if one or more power supply circuit/circuits is/are out of control and/or if the power amplifier absorption exceeds the maximum allowable current.

## Patentansprüche

1. Elektromedizinisches Hochfrequenzgerät für die Schmerztherapie und Physiotherapie, das Wellen mit zwei oder mehreren möglichen diskreten und vorherbestimmten Arbeitsfrequenzen je nach Art der auszuführenden Behandlung und Patient zur Aktivierung der Zellstimulation im Muskel- und Bindegewebe ausgibt, das mindestens einen Hochfrequenzgenerator, geeignet zum Anschluss an ein Stromversorgungsnetz, und mindestens ein Handstück mit Applikationskopf, geeignet zum Anschluss an den besagten Generator, umfasst, wobei der besagte Generator so konfiguriert ist, dass er keine Wellen mit anderen Werten als die vorherbestimmten Arbeitsfrequenzen von 2 MHz, 4 MHz und 8 MHz ausgeben kann und jeweils eine Frequenz wählt, mit einstellbarer Ausgabeleistung, mit Ausgabezyklus, dessen Dauer einstellbar ist, und mit programmierbarer/n Wiederholung oder Wiederholungen des besagten Zyklus, wobei das besagte elektromedizinische Gerät Folgendes umfasst:
• mindestens ein oder zwei der besagten Handstücke, wovon jedes mit einem entsprechenden, substantiell plattenartigen Applikationskopf ausgestattet ist, und wobei die besagten Applikationsköpfe der zwei Handstücke verschiedene Größen haben, und die Oberfläche der besagten Platte einer Verglasungsbehandlung unterzogen wird;
• mindestens eine isolierte Rückführmatte, geeignet zum elektrischen Anschluss an den besagten Generator, um den Hochfrequenz-Stromkreis zu schließen, und wobei die besagte Matte zwischen dem Patienten und der Lagerungsfläche, also dem Stuhl oder Bett, worauf der Patient positioniert ist, platziert werden muss,
und wobei die maximale Ausgabeleistung des Generators substantiell kontinuierlich ist und 100 W RMS auf eine Last von substantiell 100 Ohm beträgt.

2. Elektromedizinisches Hochfrequenzgerät gemäß eines jeden der vorstehenden Patentansprüche, **dadurch gekennzeichnet, dass** das besagte Gerät bei gleicher Leistungsausgabe eine Stimulation mit unterschiedlicher Intensität, abhängig vom verwendeten Handstück, ausführt.

3. Elektromedizinisches Hochfrequenzgerät gemäß eines jeden der vorstehenden Patentansprüche, **dadurch gekennzeichnet, dass** das besagte Gerät ein erstes Handstück mit einem 40-mm-Applikationskopf und einer Höchstintensität von ungefähr 8 Jcm2 während des normalen Gebrauchs und ein zweites Handstück mit einem 50-mm-Applikationskopf und einer Höchstintensität von ungefähr 5 Jcm2 umfasst.

4. Elektromedizinisches Hochfrequenzgerät gemäß eines jeden der vorstehenden Patentansprüche, **dadurch gekennzeichnet, dass** es auch ein weiteres Handstück mit einem 60-mm-Applikationskopf und einer Intensität von ungefähr 3,5 Jcm2 umfasst.

5. Elektromedizinisches Hochfrequenzgerät gemäß eines jeden der vorstehenden Patentansprüche, **dadurch gekennzeichnet, dass** die besagte Platte eines jeden Handstücks mit demselben Handstück abnehmbar verbunden ist.

6. Elektromedizinisches Hochfrequenzgerät gemäß eines jeden der vorstehenden Patentansprüche, **dadurch gekennzeichnet, dass** es die Aktivierung des Generators bei falscher Verbindung oder fehlender Verbindung zwischen Generator und Handstück ausschließt.

7. Elektromedizinisches Hochfrequenzgerät gemäß eines jeden der vorstehenden Patentansprüche, **dadurch gekennzeichnet, dass** der besagte Generator mit einer Mindestleistung arbeitet, wenn die besagte Platte des Handstücks das Gewebe des Patienten nicht berührt und/oder bei falscher Verbindung oder fehlender Verbindung zwischen dem besagten Generator und der besagten isolierten Rückführungsmatte.

8. Elektromedizinisches Hochfrequenzgerät gemäß eines jeden der vorstehenden Patentansprüche, **dadurch gekennzeichnet, dass** der besagte Generator automatisch und sofort deaktiviert wird, wenn ein oder mehrere Stromversorgungskreis/e außer Kontrolle ist/sind, und/oder wenn die Aufnahme des Leistungsverstärkers über dem zulässigen Höchststrom liegt.

## Revendications

1. Dispositif électro-médical à haute fréquence pour la thérapie de douleur et la physiothérapie, émettant des ondes à deux ou plusieurs fréquences di fonctionnement préétablies et discrètes possibles, selon le type de traitement à exécuter et le patient, pour l'activation de la stimulation cellulaire dans le tissu musculaire et conjonctif, comprenant au moins un générateur à haute fréquence apte à être relié à un réseau électrique d'alimentation, au moins une poignée à tête d'application apte à être reliée audit générateur, où ledit générateur est configuré de manière à ne pas émettre des ondes à des valeurs différentes des fréquences de fonctionnement préétablies de 2 MHz, 4 MHz et 8 MHz, en sélectionnant une fréquence à la fois, avec une puissance d'émission réglable, avec un cycle d'émission ayant une durée réglable et avec la répétition ou les répétitions dudit cycle programmables, et où ledit dispositif électro-médical comprend :
• au moins un ou deux desdites poignées, chacune étant dotée d'une tête d'application essentiellement aplatie, et où lesdites têtes d'application des deux poignées présentent des mesures différentes, la surface de ladite plaque étant soumise à un traitement de vitrification de surface ;
• au moins un tapis isolé de retour, apte à être relié électriquement audit générateur de manière à fermer le circuit de courant à haute fréquence, et où ledit tapis doit être placé entre le patient e la surface de support, la chaise ou le lit où le patient est installé ;
et où la puissance de sortie maximale du générateur est essentiellement continue et est égale à 100 W rms avec une charge essentiellement de 100 Ohm.

2. Dispositif électro-médical à haute fréquence selon l'une quelconque des revendications précédentes, **caractérisé en ce que**, avec une puissance d'émission égale, ledit dispositif exécute des stimulations à intensités différentes, en fonction de la poignée utilisée.

3. Dispositif électro-médical à haute fréquence selon l'une quelconque des revendications précédentes, **caractérisé en ce que** ledit dispositif comprend une première poignée à tête d'application de 40 mm avec une intensité maximale d'environ 8 Jcm2 durant l'utilisation normale, et une deuxième poignée à tête d'application de 50 mm avec une intensité maximale d'environ 5 Jcm2.

4. Dispositif électro-médical à haute fréquence selon l'une quelconque des revendications précédentes, **caractérisé en ce qu'**il comprend également une poignée supplémentaire à tête d'application de 60 mm avec une intensité d'environ 3,5 Jcm2.

5. Dispositif électro-médical à haute fréquence selon l'une des revendications précédentes, **caractérisé en ce que** ladite plaque de chaque poignée est bloquée de manière amovible à la même poignée.

6. Dispositif électro-médical à haute fréquence selon l'une quelconque des revendications précédentes, **caractérisé en ce qu'**il exclut l'activation du générateur en cas de connexion erronée ou absente entre le générateur et la poignée.

7. Dispositif électro-médical à haute fréquence selon l'une quelconque des revendications précédentes, **caractérisé en ce que** ledit générateur fonctionne au minimum de sa puissance quand ladite plaque de la poignée n'est pas en contact avec le tissu du patient et/ou en cas de connexion erronée ou absente entre ledit générateur et ledit tapis isolé de retour.

8. Dispositif électro-médical à haute fréquence selon l'une quelconque des revendications précédentes, **caractérisé en ce que** ledit générateur est automatiquement et immédiatement désactivé si un ou plusieurs circuits d'émission de puissance résultent hors contrôle et/ou si l'absorption de l'amplificateur de puissance dépasse le courant maximum admissible.
